# EUROPÄISCHE PATENTSCHRIFT

(11) **EP 0 498 062 B1**
(45) Veröffentlichungstag und Bekanntmachung des Hinweises auf die Patenterteilung: **01.09.1993**
(21) Anmeldenummer: 91121123.3
(22) Anmeldetag: 10.12.1991
(51) Int. Cl.: D04B 1/18, A61F 13/06

(54) **Aus Textilienfäden hergestelltes Flächengebilde und daraus hergestellte Gelenkbandage**
Fabric manufactured from textile threads and joint bandage manufactured therefrom
Etoffe fabriquée de fils textiles et de bandage d'articulation fabriqué à partir de celle-ci

(30) Priorität: 05.02.1991 DE 4103386
(43) Veröffentlichungstag der Anmeldung: 12.08.1992
(73) Patentinhaber: Beiersdorf Aktiengesellschaft, 20245 Hamburg (DE)
(72) Erfinder: Bodenschatz, Stefan, Dr.-Ing., W-4240 Emmerich (DE); Feldberger, Wolfgang, W-4240 Emmerich (DE); Spenke, Hans, W-4240 Emmerich (DE)
(74) Vertreter: Richter, Werdermann & Gerbaulet

(56) Entgegenhaltungen:
- DE-C- 666 955
- FR-A- 1 361 324
- FR-A- 2 633 512
- GB-A- 530 502
- GB-A- 1 352 041
- GB-A- 2 167 459
- GB-A- 2 197 351
- US-A- 4 062 204
- US-A- 4 977 011

## Beschreibung

Die Erfindung betrifft eine Gelenkbandage mit einem in deren Beugebereich angeordneten Einsatz aus einem aus textilen Fäden bestehenden Gestrick und mit einem Kompressionsbereich.

Gelenkbandagen sind in vielfacher Ausgestaltung bekannt und werden für unterschiedliche Gelenkpartien des menschlichen oder tierischen Körpers bei Gelenkverletzungen verwendet, um das Gelenk durch Kompression bzw. durch ggf. eingearbeitete zusätzliche Stabilisierungskörper zu stützen. Am häufigsten werden derartige Bandagen als Fuß-, Knie-, Hand-, Ellenbogen- und Schultergelenk-Bandagen ausgebildet.

Insbesondere bei letzteren Gelenkbandagen tritt aufgrund des dort vorliegenden, verhältnismäßig großen Beugungswinkels häufig das Problem unerwünschter Faltenbildung auf. Diesem Problem kann dadurch entgegengewirkt werden, daß die Bandage zumindest in den kritischen Beugungsbereichen mit einem höher elastischen Garn gestrickt wird. Derartige Garne sind allerdings verhältnismäßig teuer. Darüber hinaus führt die Verwendung dieser Garne dazu, daß sich im Streck- bzw. Ruhezustand des Gelenks eine zu hohe Kompression einstellt, die zu Abschnürungen der Blutgefäße führen kann und dementsprechend beim Tragen der Bandage Unannehmlichkeiten verursacht.

Die FR-A 2 633 512 beschreibt eine elastische Stütz-, Kompressions- und/oder Haltebandage, die zum Stützen, zur Kompression und zum Halten von Gelenken, wie Fuß-, Knie- und Ellenbogengelenken dient. Diese Bandage ist im wesentlichen in einer Streckbindung hergestellt, die abwechselnd Maschenreihen mit geraden und ungeraden Fangmaschen aus nicht dehnbarem Garn enthält, wobei Reihen aus Jersey-Maschen zwischen jede Reihe aus Fangmaschen eingeschoben sind. Diese Jersey-Maschen bestehen aus elastischem Garn. Eine derart ausgebildete Bandage soll ein angenehmes, ästhetisches Aussehen besitzen, eine leichte und sehr luftige Struktur aufweisen und die Schweißbeseitigung erleichtern, wobei die Bandage auf der der Haut zugewandten Seite mit einem angenehmen Tragekomfort versehen sein soll. Nach einer weiteren Ausführungsform weist diese Bandage Abschnitte bzw. Bereiche auf, die den unterschiedlichen Krümmungsbögen des Gelenkes angepaßt sind. Hierzu wird der Bereich der Bandage, der auf dem kürzesten Krümmungsbogen des Gelenkes zu liegen kommt, in herkömmlichen Maschen hergestellt, während für diejenigen Bereiche, in denen eine größere Dehnbarkeit erforderlich ist und in denen der Krümmungsbogen am längsten ist, Flächengebilde eingesetzt werden, die aus der Strickbindung hergestellt sind, die abwechselnd Maschenreihen mit geraden und ungeraden Fangmaschen aus nicht dehnbarem Garn enthält, wobei Reihen aus aus einem elastischen Garn bestehenden Jersey-Maschen zwischen jede Reihe aus Fangmaschen eingeschoben sind. Mit dieser Ausgestaltung soll einer Faltenbildung in demjenigen Bindungsbereich entgegengewirkt werden, der im Bereich des kürzesten Krümmungsbogens des Gelenkes liegt. Alternativ dazu wird das Entgegenwirken einer Faltenbildung bei dieser Gelenkbandage jedoch durch das Einbringen eines Gummischußladens in bestimmte Umfangsbereiche der Gelenkbandage erzielt. Ein einheitlich ausgebildetes Gestrick mit einer Querwellenverdichtung und einer Querwellenverringerung ist bei dieser Gelenkbandage nicht vorgesehen.

Die GB-A 530 502 beschreibt ein gekräuseltes Wirkgewebe, das im wesentlichen aus zwei unterschiedlichen Fasern besteht, die als "Bestandteile" bezeichnet werden, von denen der eine, d.h. der elastische, Bestandteil aus einem elastischen Faden und der andere, d.h. der unelastische Bestandteil, aus relativ unelastischen Fäden, wie Baumwolle oder Wolle, hergestellt ist, wobei beide Bestandteile durch Wirkverfahren in vorbestimmten Abständen miteinander verbunden werden, wobei der elastische Bestandteil in Länge und Breite gespannt wird, damit er der größeren Fläche des unelastischen Bestandteiles entspricht. Dieses gekräuselte Wirkgewebe weist unterschiedliche Kombinationen in den Verläufen der elastischen und unelastischen Fäden auf. Die Verwendung dieses gekräuselten Wirkgewebes in Form eines Einsatzes bei Gelenkbandagen, und zwar insbesondere im Beugebereich des Gelenkes, ist nicht vorgesehen, da das Wirkgewebe eine Effektstruktur aufweist, die die Eigenschaft eines Rippengestrickes nicht besitzt.

Ein Verfahren zur Herstellung einer Wirkware mit elastischer Wirkstruktur mit einem gerippten oder gerüschten Aussehen beschreibt die GB-A 2 197 351. Die nach diesem Verfahren hergestellte Wirkware kann eine einheitliche Wellen-bzw. Rippenstruktur aufweisen, die jedoch nicht für einen Einsatz bei Gelenkbandagen vorgesehen ist, um einer etwaigen Faltenbildung im inneren Gelenkbereich entgegenzuwirken.

Die DE-C 666 955 beschreibt ein Strumpfoberteil, das aus einem in Umfangsrichtung dehnbaren, oberen Rand über dem Knie in Verbindung mit einem nachgiebigen Strumpfteil in der Kniegegend besteht, der die bei einer Kniebeuge auftretende Längsdehnung des Strumpfstoffes aufnimmt, während der obere Rand des Strumpfes in seiner Lage verbleibt.

Einer Faltenbildung im Bereich des kürzesten Krümmungsbogens des Gelenkes, also z.B. auf der Innenseite eines Kniegelenkes, wird jedoch nicht entgegengewirkt. Außerdem weist das Strumpfoberteil im Bereich des größten Krümmungsbogens des Gelenkes zur Anpassung an die verschiedenen Dehnungserfordernisse ein Wellenprofil auf, das durch ein Gestrick erreicht wird, das aus abwechselnden Reihen dehnbarer und undehnbarer Maschen besteht. Die bei diesem Strumpfoberteil eingesetzte spezifische Gestrickart reicht jedoch nicht aus, um einer Faltenbildung im Bereich des kürzesten Krümmungsbogens des Gelenkes entgegenzuwirken.

Der Erfindung liegt die Aufgabe zugrunde, eine Gelenkbandage der eingangs genannten Art mit einem in deren Beugebereich angeordneten Einsatz aus einem aus textilen Fäden bestehenden Gestrick zu schaffen, die nicht nur wirtschaftlich herstellbar ist, sondern bei der selbst nach längerem Gebrauch keine Faltenbildung im Beugebereich der angelegten Gelenkbandage eintritt, wobei der Faltenbildung mit dem Gestrick bei Benutzung der Bandage entgegengewirkt werden soll, aus dem der Einsatz der Gelenkbandage besteht.

Diese Aufgabe wird durch die in dem Patentanspruch 1 gekennzeichneten Merkmale gelöst.

Durch die Merkmale einer Gelenkbandage nach dem Patentanspruch 1 wird eine Gelenkbandage geschaffen, die mit einem Einsatz aus einem Gestrick im Gelenkbeugebereich versehen ist, das zumindest auf einer Seite ein Relief in Form einer Wellenstruktur besitzt. Diese Wellenstruktur wird durch eine darunterliegende Fadenanordnung aus höherelastischem Garn bzw. Faden elastisch vorgespannt und stabilisiert, wodurch die Deckstruktur im entspannten Zustand des Gestricks wellenartig, vorzugsweise halbwellenartig ausbaucht. Wenn dementsprechend diese Flächenstruktur bei einer Gelenkbandage eingesetzt und senkrecht zur Ausrichtung der Querwellen beansprucht wird, was beispielsweise beim Beugen des bandagierten Gelenkes der Fall ist, so werden zunächst lediglich die Querwellen flacher bzw. glatt gezogen, ohne daß eine Streckung bzw. Reckung der Deckstruktur erfolgt. Überdehnungen der Deckstruktur können auf diese Art und Weise zuverlässig ausgeschlossen werden, wodurch gleichzeitig das Auftreten von Falten, und zwar selbst nach längerem Gebrauch der Bandage, im innenliegenden Gelenkbeugebereich vermieden wird. Das Gestrick eignet sich deshalb insbesondere zur Verwendung in Gelenkbandagen, die für Gelenke mit großem Bewegungsfreiraum bzw. Beugewinkel eingesetzt werden, wie das beispielsweise bei Ellenbogen, Knie- oder Schulterbandagen ist. Besonders vorteilhaft ist die Ausgestaltung nach dem Patentanspruch 2, nach der das Gestrick eine zumindest auf einer Seite ausgebildete, im wesentlichen senkrecht zur Hauptdehnungsrichtung ausgerichtete Wellenstruktur einer Deckstruktur aufweist, wobei die diese Wellenstruktur durch eine eingearbeitete oder unter dieser Deckstruktur liegende elastische Fadenanordnung elastisch vorgespannt und stabilisiert ist, die in vorbestimmten Abständen mit der Deckstruktur verbunden ist und derart geformt ist, daß die Anzahl der Querwellen dort am größten ist, wo aufgrund der Gelenkbeugung der größte Dehnungsweg auftritt.

Der erfindungsgemäße Aufbau der Gelenkbandage erlaubt somit trotz verhältnismäßig hohen Dehnungsvermögens die Verwendung von verhältnismäßig unelastischem Garn für die Deckstruktur, wodurch die Voraussetzung für eine wirtschaftliche Herstellung des textilen Flächengebildes geschaffen werden. Des weiteren besteht bei dem Gestrick keine Gefahr von überdehnungen des eingesetzten Garns, da eine große Grund-Elastizität vorhanden ist.

Vorteilhafte Ausgestaltungen der Erfindung sind in den Unteransprüchen gekennzeichnet.

Vorzugsweise wird der Einsatz aus dem Gestrick bei einer Gelenkbandage nur bereichsweise eingesetzt, nämlich dort, wo die größten Dehnungswege zu erwarten sind. Die Ausrichtung der Querwellen erfolgt regelmäßig senkrecht zur Haupt-Dehnungsrichtung. Es ist auch möglich, in dem durch die Gelenkbeugung höchst beanspruchten Bereich der Gelenkbandage mehrere textile Flächenstrukturen so anzuordnen, daß die jeweiligen Querwellen-Ausrichtungen im Winkel zueinander stehen. Diese Anordnung wird jedoch in Abstimmung mit der jeweiligen Sonderfunktion der Gelenkbandage getroffen.

Es hat sich gezeigt, daß bereits dann, wenn das Deckgestrick aus gewöhnlichem Garn, wie z.B. Baumwoll- und/oder Polyamid-Garn besteht, eine ausreichend große Elastizität des Gestricks bereit gestellt wird, um das Auftreten der eingangs angesprochenen Faltenbildung während der Lebensdauer der Bandage auszuschließen. In das Deckgestrick kann auch ein Einlegefaden eingearbeitet sein, um auch in diesem Bereich zusätzlich eine Kompressionswirkung zu erzeugen.

Wenn das Garn des elastischen Gestricks plattiert wird ergibt sich zusätzlich eine wesentlich verbesserte Verschleißfestigkeit, was sich insbesondere im Beugungsbereich der Gelenkbandage positiv auswirkt, da dort Relativbewegungen zwischen Bandage und Haut verstärkt auftreten.

Ausführungsbeispiele der Erfindung werden nachstehend anhand schematischer Zeichnungen erläutert. Es zeigen
Fig. 1 bis 4 verschiedene Anordnungen des Gestricks in unterschiedlichen Gelenkbandagen,
Fig. 5 eine Einzelheit des Schnitts V-V in Fig. 2,
Dif. 6 in schematisch vereinfachter Darstellung das Prinzip der Verformung eines aus dem Gestrick bestehenden Flächengebildes,
Fig. 7 bis 9 weitere Ausführungsformen von Gelenkbandagen,
Fig. 10 Darstellungen von Strickreihen zur Verdeutlichung einer ersten Ausführungsform des Verfahrens zur Herstellung des Gestricks, und
Fig. 11 in einer der Fig. 10 ähnlichen Darstellung ein weiteres Strickmuster für das Gestrick.

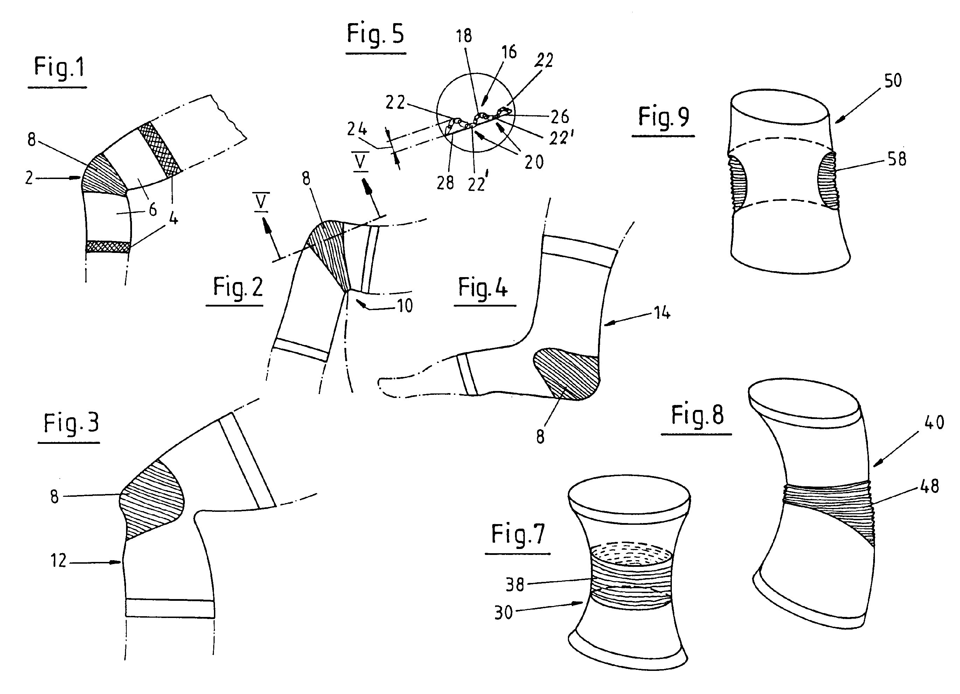

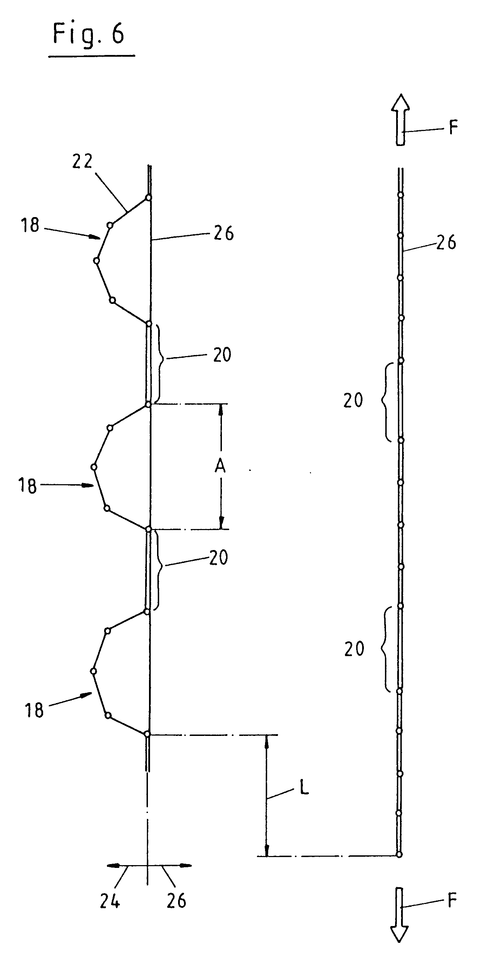

In Fig. 1 ist die Gelenkpartie im Bereich eines Ellenbogens gezeichnet, die von einer Ellenbogen-Gelenkbandage 2 stabilisiert ist. Es handelt sich hierbei um eine elastische Bandage in Schlauchform, d.h. eine sogenannte Kompressionsbandage, wie sie bei Gelenkverletzungen bzw. Gelenkschwächen Verwendung findet. Die Bandage setzt sich im wesentlichen aus drei Abschnitten zusammen: Randseitig ist ein Bundabschnitt 4 vorgesehen, um eine rutschfeste Fixierung der Bandage 2 zu gewährleisten. Daran schließt sich der eigentliche Kompressionsbereich 6 an, der über das Gelenk hinwegläuft. Im Kompressionsbereich 6 ist ein Einsatz aus einem Gestrick 8 eingearbeitet, und zwar dort, wo aufgrund der Gelenkbeugungen größte Dehnungen der Bandage 2 auftreten.

In den Fig. 2 bis 4 sind weitere Gelenkbandagen, nämlich eine Schultergelenkbandage 10 (Fig.2), eine Kniegelenkbandage 12 (Fig.3) und eine Fußgelenkbandage 14 (Fig.4) dargestellt, die ähnlich wie die Ellenbogen-Gelenkbandage 2 aufgebaut sind und jeweils im Kompressionsbereich mit Einsätzen aus dem Gestrick 8 ausgestattet sind. Diese Einsätze sind in den Figuren mit Längsstreifen gekennzeichnet.

Bei dem gezeigten Ausführungsbeispiel bestehen die Bandagen 2 und 10 bis 14 aus Strickware, wobei für die einzelnen Abschnitte 4, 6 und 8 unterschiedliche Strickarten Anwending finden. Es sind allerdings auch andere Textilwaren und/oder Maschenwaren verwendbar.

Die Besonderheit der gezeigten Bandagen besteht darin, daß für die Einsätze, die dort vorgesehen sind, wo die höchsten Dehnungs-Wechselbeanspruchungen der Bandage auftreten, eine besondere Textilstruktur, insbesondere ein besonderes Gestrick 8, verwendet wird, welches nachstehend näher erläutert wird.

Die streifenförmige Hinterlegung der Einsätze deutet an, daß das Gestrick 8 in diesem Bereich ein Relief bildet, welches aus dem Schnitt V-V in Fig.2 in der Darstellung gemäß Fig.5 verdeutlicht wird. Bei diesem Relief handelt es sich um eine Wellenstruktur, die zumindest auf einer Seite der Bandage - bei der gezeigten Ausführungsform auf der dem Körper abgewandten Seite - ausgebildet wird. Dabei ist eine Reihe von Halbwellen 18 unter Zwischenschaltung von Knotenpunkten bzw. Knotenbereichen 20 aneinandergereiht, die wie folgt entstehen:

Ein von Maschen 22 gebildetes Deckgestrick 24 ist im Bereich einzelner Maschen 22' mit einer elastischen Fadenanordnung 26 auf der Unterseite des Deckgestricks 24 derart fest verbunden, daß eine Reihe von Maschen 22 des Deckgestricks 24 - bei der Ausführungsform vier - von einer längeren Masche 28 der darunterliegenden, elastischen Fadenanordnung 26 überbrückt wird. Die Maschen 22 zwischen den Knotenpunkten bzw. -bereichen 20 werden dadurch nach oben ausgebaucht, wodurch die Querwellen 18 vorgespannt und stabilisiert werden. Durch Variation der Anzahl der überbrückten Maschen 22 und/oder der Maschen 28 kann auf das Verformungsverhalten und die Dauerelastizität gezielt Einfluß genommen werden.

Das Verformungsverhalten des auf diese Art und Weise gebildeten Gestricks 8 geht im einzelnen aus der Darstellung gemäß Fig.6 hervor. Auf der linken Seite ist das Gestrick im entlasteten Zustand gezeigt. Die Maschen sind durch Linien angedeutet und die Verbindung der Maschen untereinander durch kleine Kreise.

Das auf der Unterseite vorgesehene elastische Garn 26 überbrückt die Knotenbereiche 20, zwischen denen jeweils vier Maschen 22 des Deckgestricks 24 ausgebildet sind. Durch die Vorspannung der elastischen Fadenanordnung 26 werden die Maschen 22 zu Halbwellen 18 ausgebaucht, wodurch die Reliefstruktur entsteht. Die Halbwellen 18 weisen jeweils zwischen zwei und zwölf, vorzugsweise vier, Maschenreihen auf.

Auf der rechten Seite ist dargestellt, wie sich das Gestrick 8 bei Beanspruchung durch eine Zugkraft F verhält. Es ist erkennbar, daß die elastische Fadenanordnung 26 zwischen den Knotenbereichen 20 gedehnt wird, ohne daß eine zusätzliche Dehnungsbeanspruchung im Bereich der Halbwellen 18, d.h. im Bereich der Maschen 22, auftritt. Das Gestrick 8 kann sich dementsprechend um das Maß L längen, bevor die Maschen 22 des Deckgestricks 24 auf Zug beansprucht werden. Dieses Maß L schafft dementsprechend eine Dehnungsreserve des Bandagengestricks im Vergleich zu herkömmlichen textilen Flächengebilden.

Die Verbindung zwischen Deckgestrick 24 und elastischer Fadenanordnung 26 kann auf verschiedenste Art und Weise erfolgen. Es ist auch möglich, die Verbindung derart zu wählen bzw. herzustellen, daß auf beiden Seiten des Gestricks 8 Wellen gebildet werden. Das Deckgestrick 24 braucht nicht einflächig ausgebildet zu sein.

Durch die vorstehende Struktur des Gestricks 8 kann trotz Bereitstellung einer hohen Dehnungselastizität für das Deckgestrick normales Strickgarn, wie z.B. Baumwolle oder Polyamidgarn, verwendet werden. Für die elastische Fadenanordnung 26 wird vorzugsweise höherelastisches Garn, wie z.B. Umwindegarn, verwendet, wobei dieser elastische Faden zusätzlich plattiert werden kann, um die Verschleißfestigkeit des textilen Gewebes zu verbessern.

In die Maschen 22 des Deckgestricks 24 kann ferner ein Einlegefaden eingearbeitet werden, um auch in diesem Bereich der wellenförmigen Reliefstruktur eine Kompressionswirkung der Bandage zu erreichen.

Das auf diese Art und Weise geschaffene Wellengestrick kann in die Bandage an den verschiedensten Stellen und in unterschiedlichster Formgebung eingesetzt werden, was durch die Fig. 7 bis 9 beispielsweise angedeutet werden soll. Das Gestrick 8 kann auch zusätzlich beispielsweise mit elastischem Garn abgesteppt werden.

Bei der in Fig. 7 gezeigten schlauchförmigen Gelenkbandage 30 erstreckt sich der Einsatz aus dem elastischen Gestrick 38 über den gesamten Umfang in gleichmäßiger Höhe.

Bei der Bandage 40 gemäß Fig. 8 verläuft der Einsatz mit dem Gestrick 48 zwar auch ringförmig um die schlauchförmige Bandage herum, hat jedoch auf einer Seite eine größere Erstreckung als auf der anderen Seite.

Die Bandage 50 gemäß Fig. 9 schließlich weist einen Einsatz mit einem Gestrick 58 auf, der sich mit im wesentlichen gleichförmiger Breite über einen Zentrierwinkel von etwa 180° um die Bandage herum erstreckt. Die Gestricke 8,38,48,58 sind gleich ausgebildet.

Abweichend von den dargestellten Ausführungsbeispielen ist es auch möglich, im Beugungsbereich der Gelenkbandagen mit mehreren unterschiedlich orientierten Einsätzen zu arbeiten, um auf diese Weise der spezifischen Dehnungsbeanspruchung der Bandage Rechnung zu tragen.

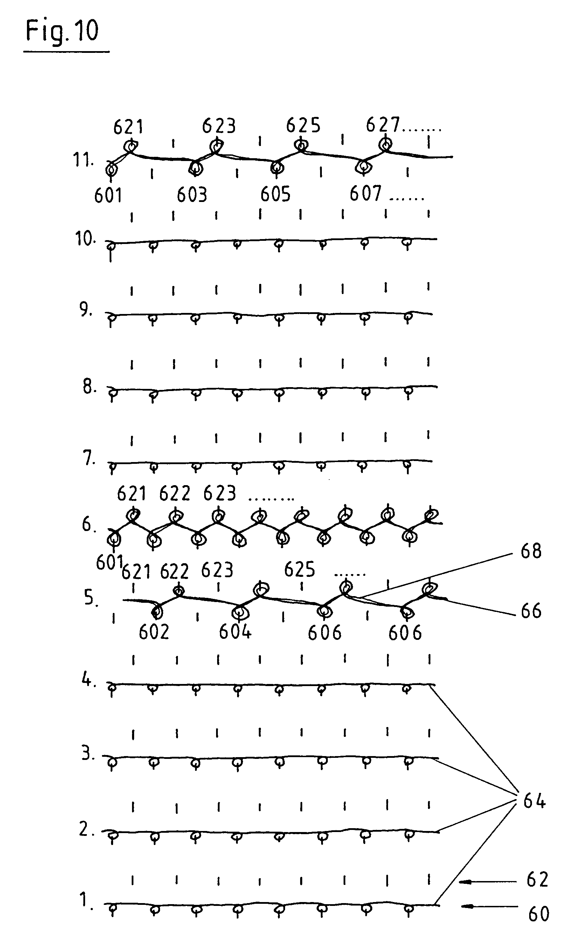

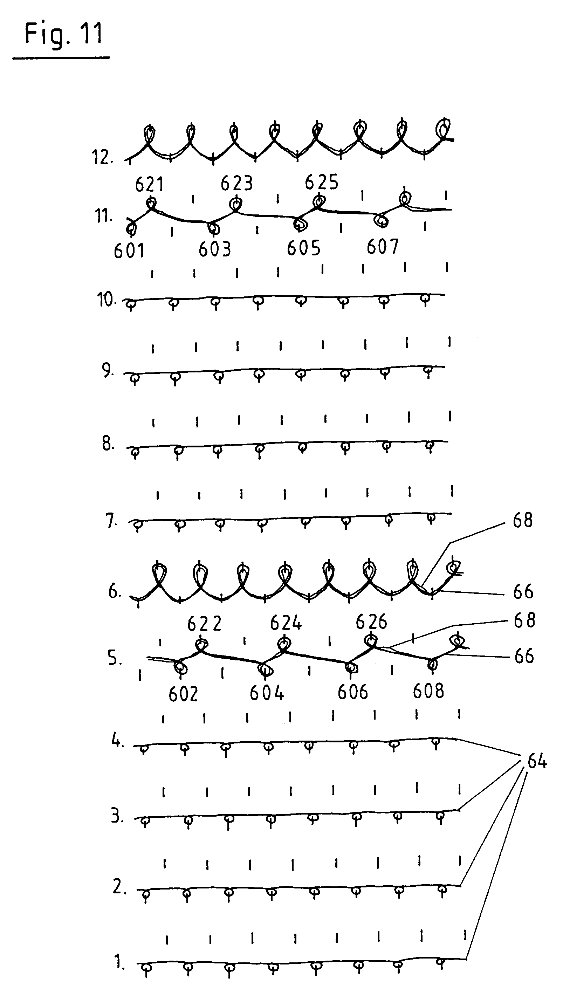

In den Fig. 10 und 11 sind zwei Möglichkeiten aufgezeigt, wie das vorstehend erläuterte Gestrick 8 auf automatischen Strickmaschinen hergestellt werden kann. Hierbei werden zwei Nadelbetten verwendet, nämdlich ein erstes Nadelbett 60 und ein zweites Nadelbett 62 mit im gleichen Abstand zueinander angeordneten Nadeln. Gestrickt wird auf dem ersten Nadelbett 60 mehrere - im gezeigten Ausführungsbeispiel vier - Maschenreihen aus normalem Strickgarn, wie z.B. Baumwolle- oder Polyamidgarn 64. Anschließend werden zwei Maschenreihen auf beiden Nadelbetten 60,62 mit elastischem Garn, wie z.B. Gummi oder Umwindegarn 68 gestrickt, wobei diesem elastischen Garn vorzugsweise ein Plattierfaden 66 beigegeben wird. Bei der fünften Strickreihe wird nur auf ausgewählten Nadeln 602,604,606,... usw. bzw. 622, 624,626,...usw. beider Nadelbetten gestrickt. Der Plattierfaden kann von einem Polyamid HE-Faden gebildet sein. Die Darstellung läßt erkennen, daß die Maschenreihe sechs wieder auf allen Nadeln der beiden Nadelbetten 60,62 gestrickt wird. Dies ist jedoch nicht unbedingt erforderlich.

Es folgen dann wiederum vier Maschenreihen mit gewöhnlichem Garn auf dem ersten Nadelbett 60 und abschließend zwei weitere Maschenreihen unter Verwendung elastischen Garns, wobei sich die elfte Strickreihe von der fünften dadurch unterscheidet, daß die beteiligten Nadeln der Nadelbetten 60,62 um eins versetzt sind.

Fig. 11 zeigt ein anderes Muster mit einer etwas anderen Bindung im Bereich der späteren Gestrick-Knoten 20. Die Strickreihen eins bis fünf und sieben bis elf entsprechen dem Strickmuster gemäß Fig. 10. Unterschiedlich ist lediglich die Ausbildung der Strickreihen 6 bzw. 12, so daß ein näheres Eingehen auf diese Figur nicht erforderlich erscheint.

Abweichend von dem zuvor beschriebenen Herstellungsverfahren ist es auch möglich, zunächst auf einem Nadelbett eine oder mehrere Maschenreihen aus elastischem Garn, wie z.B. Umwindegarn, zu stricken und auf dem anderen Nadelbett Maschenreihen aus normalem Strickgarn , wie z.B. Baumwolle- oder Polyamidgarn, wobei anschließend eine oder mehrere Maschenreihen mit allen oder einzelnen Nadeln beider Nadelbetten gestrickt werden.

Entscheidend ist, daß das textile Flächengebilde einen solchen Aufbau erhält, daß eine eingearbeitete und nach vorbestimmtem Muster mit einer textilen Deckstruktur verbundene elastische Fadenanordnung dem Flächengebilde eine solche innere Vorspannung gibt, daß zumindest auf einer Seite ein wellenartiges Relief entsteht, das dann durch äußere Beanspruchung zunächst glatt bzw. glatter gezogen werden kann, ohne bereits in dieser Phase die Deckstruktur auf Dehnung zu beanspruchen.

Die Erfindung schafft somit ein aus textilen Fäden hergestelltes Gestrick zur Verwendung im Beugebereich von Gelenkbandagen. Zur Eliminierung von Faltenbildung im Beugebereich der Gelenkbandage wird zumindest auf einer Seite eine Querwellenstruktur ausgebildet, die durch eine maschenreihenweise eingearbeitete und unter einer Deckstruktur liegende elastische Fadenanordnung, die in vorbestimmten Abständen bzw. nach einer bestimmten Regel mit der Deckstruktur verbunden ist, elastisch vorgespannt und stabilisiert ist.

## Patentansprüche

1. Gelenkbandage mit einem in deren Beugebereich angeordneten Einsatz aus einem aus textilen Fäden bestehenden Gestrick (8,38,48,58) und mit einem Kompressionsbereich (6), dadurch gekennzeichnet, daß das Gestrick (8,38,48,58) eine zumindest auf einer Seite ausgebildete, im wesentlichen senkrecht zur Hauptdehnungsrichtung (F) ausgerichtete Wellenstruktur (16,18) einer Deckstruktur aufweist, wobei die Wellenstruktur (16, 18) durch eine maschenreihenweise eingearbeitete und unter dieser Deckstruktur liegende elastische Fadenanordung (26) elastisch vorgespannt und stabilisiert ist, die in vorbestimmten Abständen (A) mit der Deckstruktur (16,18) verbunden ist.

2. Gelenkbandage nach Anspruch 1, dadurch gekennzeichnet, daß das Gestrick (8,38,48,58) derart geformt ist, daß die Anzahl der Querwellen (18) dort am größten ist, wo aufgrund der Gelenkbeugung der größte Dehnungsweg (L) auftritt.

3. Gelenkbandage nach einem der Ansprüche 1 und 2, dadurch gekennzeichnet, daß die Wellenstruktur (16,18) aus Halbwellen (18) besteht, die jeweils zwischen zwei und zwölf, vorzugsweise vier, Maschenreihen aufweisen.

4. Gelenkbandage nach einem der Ansprüche 1 bis 3, dadurch gekennzeichnet, daß das Deckgestrick (24) des Gestrickes (8) aus einer im Vergleich zur eingearbeiteten oder darunterliegenden Fadenstruktur weniger elastischen Maschenware (64) mit unelastischen Fäden besteht.

5. Gelenkbandage nach einem der Ansprüche 1 bis 4, dadurch gekennzeichnet, daß das Deckgestrick (24) aus Baumwoll- und/oder Polyamid-Garn besteht.

6. Gelenkbandage nach einem der Ansprüche 1 bis 5, dadurch gekennzeichnet, daß die elastische Fadenanordnung (26) aus Umwindegarn (68) besteht oder dieses aufweist.

7. Gelenkbandage nach Anspruch 6, dadurch gekennzeichnet, daß das Umwindegarn (68) plattiert ist.

8. Gelenkbandage nach einem der Ansprüche 1 bis 7, dadurch gekennzeichnet, daß in das Deckgestrick (24) ein Einlegefaden eingearbeitet ist.

9. Gelenkbandage nach einem der Ansprüche 1 bis 8, dadurch gekennzeichnet, daß in dem durch Gelenkbeugung höchstbeanspruchten Bereich der Gelenkbandage mehrere textile Gestricke (8) angeordnet sind, wobei die jeweiligen Querwellen-Ausrichtungen im Winkel zueinander stehen.

10. Gelenkbandage nach einem der Ansprüche 1 bis 9, dadurch gekennzeichnet, daß das Gestrick (8) im Kompressionsbereich (6) der Gelenkbandage (2) dort angeordnet ist, wo aufgrund der Gelenkbeugungen größte Dehnungen der Gelenkbandage (2) auftreten.

11. Gelenkbandage nach einem der Ansprüche 1 bis 10, dadurch gekennzeichnet, daß die Gelenkbandage (2) einen ringförmigen Einsatz aus dem Gestrick (48) aufweist, wobei auf einer Seite der Gelenkbandage (2) eine größere Erstreckung als auf der anderen Seite vorgesehen ist.

12. Gelenkbandage nach einem der Ansprüche 1 bis 10, dadurch gekennzeichnet, daß die Gelenkbandage einen Einsatz aus einem Gestrick (58) aufweist, der sich mit gleichförmiger Breite über einen Zentrierwinkel von etwa 180° um die Gelenkbandage (2) herum erstreckt.

## Claims

1. Joint bandage with an inset disposed within its bending area of a fabric manufactured from textile threads (8, 38,48,58) and having a compression area (6), characterized in that the fabric (8,38,48,58) possesses a wavy structure (16,18) of a covering structure constructed at least on one side and essentially aligned vertically to the main elongation direction (F), in which the wavy structure (16,18) is elastically pretensioned and stabilized by means of an elastic thread arrangement (26) incorporated into a stich course-like manner and located underneath said covering structure, which is connected at predetermined intervals (A) to the covering structure (16,18).

2. Joint bandage according to Claim 1, characterized in that the fabric (8,38,48,58) is configured in such a way that the number of transversal waves (18) is greatest where, owing to the bending of the joint, the greatest elongation path (L) occurs.

3. Joint bandage according to either Claim 1 or 2, characterized in that the wavy structure (16,18) is comprised of half-waves which in each case have two and twelve, by preference four, stitch courses.

4. Joint bandage according to any of claims 1 to 3, characterized in that the covering fabric (24) of the fabric (8) is manufactured from a, in comparison with the incorporated or subjacent thread structure, less elastic knitted fabric (64) possessing inelastic threads.

5. Joint bandage according to any of Claims 1 to 4, characterized in that the covering fabric (24) is comprised of cotton and/or polyamide yarn.

6. Joint bandage according to any of Claims 1 to 5, characterized in that the elastic thread arrangement (26) is comprised of covered yarn (68) or is comprised of the same.

7. Joint bandage according to Claim 6, characterized in that the covered yarn (68) is plated.

8. Joint bandage according to any of Claims 1 to 7, characterized in that, a laid-in thread is incorporated into the covering fabric (24).

9. Joint bandage according to any of Claims 1 to 8, characterized in that, within the area most highly stressed by the bending of the joint of the joint bandage, several textile fabrics (8) are disposed, in which case the respective transversal wave alignments are at an angle to each other.

10. Joint bandage according to any of Claims 1 to 9, characterized in that the fabric (8), within the compression area (6) of the joint bandage (2),is disposed where, due to the bending of the joint, the greatest elongation of the joint bandage (2) takes place.

11. Joint bandage according to any of Claims 1 to 10, characterized in that the joint bandage (2) possesses an annular inset of the fabric (48) while, on the one side of the joint bandage (2), a greater extension is provided than on the other side.

12. Joint bandage according to any of Claims 1 to 10, characterized in that the joint bandage is provided with an inset of a fabric (58) that extends with a uniform width across a centering angle of approximately 180° around the joint bandage (2).

## Revendications

1. Bandage d'articulation avec un insert placé dans sa zone de flexion constitué par un tricotage (8, 38, 48, 58) en fils textiles et avec une zone de compression (6), **caractérisé en ce** que le tricotage (8, 38, 48, 58) présente une structure ondulée (16, 18) d'une structure de recouvrement configurée au moins sur un côté et orientée essentiellement perpendiculairement au sens d'allongement principal (F), la structure ondulée (16, 18) étant précontrainte élastiquement et stabilisée par un arrangement de fils (26) élastique, incorporé rangée de mailles par rangée de mailles, et se trouvant sous cette structure de recouvrement, arrangement qui est relié à des intervalles prédéterminés (A) à la structure de recouvrement (16, 18).

2. Bandage d'articulation selon la revendication 1, **caractérisé en ce** que le tricotage (8, 38, 48, 58) est formé de telle manière que le nombre d'ondes transversales (18) est le plus grand là où intervient le plus grand parcours d'allongement (L) en raison de la flexion de l'articulation.

3. Bandage d'articulation selon l'une des revendications 1 et 2, **caractérisé en ce** que la structure ondulée (16, 18) est constituée par des demi-ondes (18) qui présentent chacune entre deux et douze, et de préférence quatre rangées de mailles.

4. Bandage d'articulation selon l'une des revendication 1 à 3, **caractérisé en ce** que le tricotage de recouvrement (24) du tricotage (8) est constitué par un tissu maillé (64) avec des fils non élastiques qui est moins élastique qu'une structure de fils incorporée ou sous-jacente.

5. Bandage d'articulation selon l'une des revendications 1 à 4, **caractérisé en ce** que le tricotage de recouvrement (24) est constitué par du fil de coton et/ou de polyamide.

6. Bandage d'articulation selon l'une des revendications 1 à 5, **caractérisé en ce** que l'arrangement de fils élastiques est constitué par du fil de guipage (68) ou présente celui-ci.

7. Bandage d'articulation selon la revendication 6, **caractérisé en ce** que le fil de guipage (68) est vanisé.

8. Bandage d'articulation selon l'une des revendications 1 à 7, **caractérisé en ce** qu'un fil d'insertion est incorporé au tricotage de recouvrement (24).

9. Bandage d'articulation selon l'une des revendications 1 à 8, **caractérisé en ce** que plusieurs tricotages textiles (8) sont placés dans la zone du bandage d'articulation qui est la plus sollicitée par la flexion de l'articulation. les orientations des demi-ondes respectives formant un angle les unes par rapport aux autres.

10. Bandage d'articulation selon l'une des revendications 1 à 9, **caractérisé en ce** que le tricotage (8) est placé dans la zone de compression (6) du bandage d'articulation (2) là où interviennent les plus grands allongements du bandage d'articulation (2) en raison des flexions de l'articulation.

11. Bandage d'articulation selon l'une des revendications 1 à 10, **caractérisé en ce** que le bandage d'articulation (2) présente un insert de forme annulaire constitué par un tricotage (48), une extension plus grande étant prévue sur l'un des côtés du bandage d'articulation que sur l'autre côté.

12. Bandage d'articulation selon l'une des revendications 1 à 10, **caractérisé en ce** que le bandage d'articulation présente un insert constitué par un tricotage (58) qui s'étend avec une largeur uniforme autour du bandage d'articulation (2) par un angle de centrage d'environ 180°.
